# EUROPÄISCHE PATENTSCHRIFT

(11) **EP 1 050 276 B1**
(45) Veröffentlichungstag und Bekanntmachung des Hinweises auf die Patenterteilung: **21.12.2005**
(21) Anmeldenummer: 00107800.5
(22) Anmeldetag: 12.04.2000
(51) Int. Cl.: A61B 17/86, A61B 17/70

(54) **Pedikelschraube**
Pedicle screw
Vis pédiculaire

(30) Priorität: 06.05.1999 DE 19922440
(43) Veröffentlichungstag der Anmeldung: 08.11.2000
(73) Patentinhaber: DePuy Spine Sàrl, 2400 Le Locle (CH)
(72) Erfinder: DePuy Spine Sàrl, 2400 Le Locle (CH)
(74) Vertreter: Dreiss, Fuhlendorf, Steimle & Becker

(56) Entgegenhaltungen:
- EP-A- 0 201 905
- WO-A-94/16634
- US-A- 1 028 795
- US-A- 4 041 939
- US-A- 5 562 735

## Beschreibung

Die Erfindung betrifft eine Pedikelschraube mit den Merkmalen des Oberbegriffs des Anspruchs 1.

Pedikelschrauben sind in mannigfaltigen Ausgestaltungen bekannt. In der Regel weisen die Pedikelschrauben einen dem Knochendurchmesser in der Länge und im Durchmesser angepassten Schraubenschaft auf, welcher mit einem Gewinde versehen ist, so dass die Pedikelschraube problemlos und sicher und vor allem ohne Zerstörung des Knochens in diesen eingeschraubt werden kann. Der Schraubenkopf ist so ausgebildet, dass an diesem Korrektur- und/oder Fixierstäbe spielfrei befestigt werden können, wodurch die Osteosynthese erzielt wird.

Es hat sich gezeigt, dass bei allen Pedikelschrauben die Gefahr besteht, insbesondere bei Personen mit Osteoporose, dass die Schrauben zwar problemlos in den Knochen eingeschraubt werden können, die Schrauben jedoch entweder selbsttätig sich lockern oder bei großen äußeren Krafteinwirkungen bisweilen sogar aus dem Knochen ausgerissen werden.

Aus der US-A-4,041,939 ist eine Pedikelschraube bekannt, auf deren freies Ende eine Mutter als Ausreißsicherung aufgeschraubt werden kann. Als nachteilig wird dabei angesehen, dass das freie Ende mit einem exakten Maschinengewinde versehen werden muss und dass die Mutter eine entsprechende Gewindebohrung aufweisen muss. Diese Ausgestaltungen sind teuer und das Aufschrauben bereitet bei beengten Platzverhältnissen Schwierikeiten.

Der Erfindung liegt daher die Aufgabe zugrunde, eine Pedikelschraube der eingangs genannten Art derart weiterzubilden, dass diese mit Sicherheit im Knochen verankerbar ist und die Gefahr des Ausreißens wesentlich vermindert ist, wobei die Kosten der Schraube nur unwesentlich höher sind.

Diese Aufgabe wird erfindungsgemäß mit einer Pedikelschraube gelöst, die die Merkmale des Anspruchs 1 aufweist.

Aufgrund des Sicherungsmittels, welches am freien Ende des Gewindeschaftes der erfindungsgemäßen Pedikelschraube dann befestigt wird, wenn die Schraube ihre endgültige Position im Knochen eingenommen hat, wird die Gefahr des Ausreißens aus dem Knochen wesentlich minimiert, da die Pedikelschraube nicht nur über das Gewinde, sondern auch über das Sicherungsmittel eine Abstützung am Knochen, insbesondere an hochtorakalen Wirbeln erfährt. Das Sicherungsmittel liegt dabei auf der dem Schraubenkopf gegenüberliegenden Seite am Knochen an und der Knochen wird zwischen dem Schraubenkopf und dem Sicherungsmittel aufgenommen. Dabei weist die Sicherungsscheibe einen die Halteeinrichtung aufnehmenden Durchbruch auf. In diesem Durchbruch wird das freie Ende des Schraubenschaftes gehalten, wobei die Abmessungen des Durchbruches so gewählt sind, dass der Rand des Durchbruches z.B. in einer Umfangsrille oder in der Gewindenut liegt. Ein einfaches Befestigen des Sicherungsmittels wird dadurch erreicht, dass der Durchbruch randoffen ist, d.h. einen randoffenen Schlitz aufweist. Dabei kann der randoffene Schlitz eine geringfügig kleinere lichte Weite aufweisen, als der Durchmesser des freien Endes des Schraubenschaftes.

Bei einer Weiterbildung ist vorgesehen, dass die Halteeinrichtung in Form von wenigstens einer Umfangsrille ausgestaltet ist. Das Sicherungsmittel stützt sich an dieser Umfangsrille am Schraubenschaft ab und wird von diesem über die Umfangsrille sicher gehalten. Die Umfangsrille hat zudem den Vorteil, dass das Sicherungsmittel über den gesamten Umfang des Schaftes an diesem fixierbar ist.

Eine Weiterbildung sieht vor, dass die Halteeinrichtung ein weiteres Gewinde ist. Dieses Gewinde kann eine andere Teilung und/oder eine andere Steigung als das Einschraubgewinde des Schraubenschaftes aufweisen. In der Regel ist dieses weitere Gewinde ein Maschinengewinde, so dass an diesem Normteile, bzw. Sicherungsmittel mit einem genormten Innengewinde fixierbar sind. Außerdem sind derartige Gewinde relativ einfach herstellbar.

Eine preiswerte Ausführung des Sicherungsmittels ist z.B. eine Sicherungsscheibe, die als Stanzteil bzw. als Stanz /Biegeteil herstellbar ist. Eine Sicherungsscheibe hat außerdem den Vorteil, dass sie großflächig an der Außenfläche des Knochens anliegt und dadurch die Haltekraft optimal verteilt wird.

Um Lastspitzen opimal entgegenwirken zu können, ist das Sicherungsmittel im Verbindungsbereich mit der Halteeinrichtung elastisch ausgebildet. Auf diese Weise wird eine starre und gegen Versagen anfällige Konstruktion vermieden und die elastische Ausgestaltung hat zudem den Vorteil, dass, nach dem Überwinden der Lastspitze, das Sicherungsmittel wieder seine Ausgangslage bzw. Ruhelage einnimmt, indem es elastisch zurückfedert.

Dabei kann bei einem bevorzugten Ausführungsbeispiel die elastische Ausbildung eine Abkröpfung sein. Hierfür ist das Sicherungsmittel bevorzugt aus einem Federstahl gefertigt.

Eine bevorzugte Weiterbildung sieht vor, dass das Sicherungsmittel auf die Halteeinrichtung aufklipsbar ist. Diese Ausgestaltung erlaubt es, nach dem Einschrauben der Pedikelschraube, auf relativ einfache Art und Weise, dass das Sicherungsmittel am freien Ende des Schraubenschaftes befestigt werden kann, indem es lediglich aufgedrückt bzw. aufgepresst wird, wobei es dann bevorzugt mit dem freien Ende des Schraubenschaftes verrastet.

Um eine gewisse Elastizität des Sicherungsmittels zu erreichen, weist dieses einen oder mehrere Schlitze auf. Zwischen den Schlitzen sind Flügel ausgebildet, welche radial nach innen abstehen und mit ihren freien Enden am Schraubenschaft anliegen.

Eine Weiterbildung sieht vor, dass das Sicherungsmittel Öffnungen oder Durchbrüche aufweist, an welchen ein Werkzeug ansetzbar ist. Mittels dieses Werkzeugs kann das Sicherungsmittel befestigt und/oder entfernt werden. Vorzugsweise wird dabei eine Schraubbewegung ausgeführt.

Bevorzugte Materalien für das Sicherungsmittel sind Metalle, insbesondere Federstahl, Edelstahl oder Titan, oder das Sicherungsmittel besteht aus Kunststoff bzw. einem Verbundmaterial.

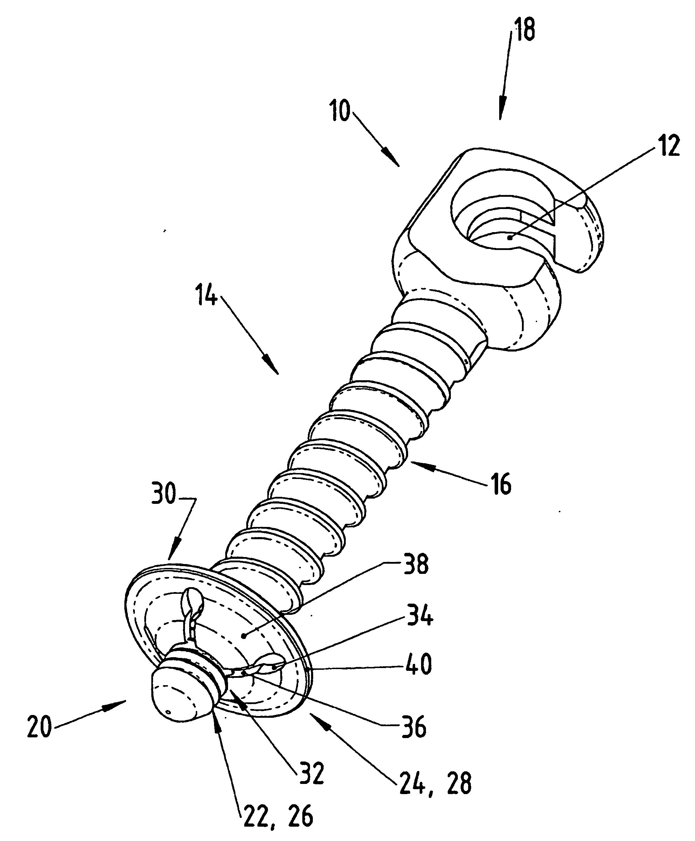

Weitere Vorteile, Merkmale und Einzelheiten ergeben sich aus der nachfolgenden Beschreibung, in der, unter Bezugnahme auf die Zeichnung, ein besonders bevorzugtes Ausführungsbeispiel im Einzelnen beschrieben ist. Dabei können die in der Zeichnung dargestellten sowie in den Ansprüchen und in der Beschreibung erwähnten Merkmale jeweils einzeln für sich oder in beliebiger Kombination erfindungswesentlich sein. Die Zeichnung zeigt eine perspektivische Ansicht der erfindungsgemäßen Pedikelschraube mit am freien Ende befestigtem Sicherungsmittel.

Die in der Zeichnung dargestellte Pedikelschraube weist einen mit 10 bezeichneten Schraubenkopf, der als Gabelkopf mit randoffenem Durchbruch 12 ausgebildet ist, und einen Schraubenschaft 14 auf, der ein erstes Gewinde 16 trägt, über welches die Pedikelschraube 18 in einen Knochen, insbesondere einen hochtorakalen Wirbel, eingeschraubt wird. Der Schraubenschaft 14 ist an seinem freien Ende 20 mit einer insgesamt mit 22 bezeichneten Halteeinrichtung für ein Sicherungsmittel 24 ausgestattet. Das freie Ende 22 ist beim dargestellten Ausführungsbeispiel mit einem vom Gewinde 16 unabhängigen weiteren Gewinde 26, welches bevorzugterweise ein Maschinengewinde ist, ausgebildet. Auf dieses Gewinde 26 ist das Sicherungsmittel 24, welches als Sicherungsscheibe 28 ausgebildet ist, aufgeschraubt. Diese Sicherungsscheibe 28 weist eine, an einem (nicht dargestellten) Wirbel anliegende Anlagefläche 30 auf, und ist mit einem zentralen Durchbruch 32 versehen, durch welches das freie Ende 20 des Schraubenschaftes 14 hindurchgeführt ist. Außerdem weist die Sicherungsscheibe 28 mehrere Löcher 34 sowie von den Löchern 34 ausgehende, sich radial nach innen erstreckende Schlitze 36 auf, die in den zentralen Durchbruch 32, einmünden. Zwischen den Schlitzen 36 werden Flügel 38 gebildet, die vom Rand 40 der Sicherungsscheibe 28 radial nach innen abstehen. Diese Flügel 38 sind derart abgekröpft, dass der radial weiter innen liegende Bereich vom (nicht dargestellten) Wirbel einen größeren Abstand aufweist, als der Rand 40, d.h. am Wirbel nicht unbedingt anliegt. Auf diese Weise wird eine gewisse Elastizität bzw. elastische Ausgestaltung der Sicherungsscheibe 28 geschaffen.

Die Löcher 34 und ggf. die Schlitze 36 dienen zum Ansetzen eines Werkzeuges, mit dem die Sicherungsscheibe 28 auf das Gewinde 26 aufgeschraubt bzw. von diesem abgeschraubt werden kann. Es besteht jedoch auch die Möglichkeit, die Sicherungsscheibe 28 mittels eines Werkzeugs, z.B. mittels einer Zange oder dergl., auf das freie Ende 20 aufzupressen, so dass die Sicherungsscheibe 28 mit dem Gewinde 26 verrastet. Die auf dem Gewinde 26 fixierte Sicherungsscheibe 28 dient als Ausreißschutz für die Pedikelschraube 18, so dass mit dieser wesentlich höhere Kräfte auf den Wirbel übertragen werden können.

## Patentansprüche

1. Pedikelschraube mit einem ein Gewinde (16) aufweisenden Schraubenschaft (14) und einem Schraubenkopf (10), wobei der Schraubenkopf (10) Einrichtungen zur Fixierung von Korrekturelementen aufweist, und das freie Ende (20) des Schraubenschaftes (14) mit einer Ausreisssicherung versehen ist, die eine Sicherungsscheibe (28) und eine Halteeinrichtung (22) für die die Pedikelschraube nach dem Einschrauben gegen unbeabsichtigtes Lösen sichernde Sicherungsscheibe (28) umfaßt, wobei die Sicherungsscheibe (28) einen die Halteeinrichtung (22) aufnehmenden Durchbruch (32)aufweist, **dadurch gekennzeichnet, dass** der Durchbruch (32) randoffen ist und einen in den Durchbruch (32) einmündendar Schlitz (36) aufweist.

2. Pedikelschraube nach Anspruch 1, **dadurch gekennzeichnet, dass** die Halteeinrichtung (22) vom Gewinde (16) unabhängig ist.

3. Pedikelschraube nach einem der vorhergehenden Ansprüche, **dadurch gekennzeichnet, dass** die Halteeinrichtung (22) in Form von wenigstens einer Umfangsrille ausgestaltet ist.

4. Pedikelschraube nach einem der vorhergehenden Ansprüche, **dadurch gekennzeichnet, dass** die Halteeinrichtung ein weiteres Gewinde (26) ist.

5. Pedikelschraube nach einem der vorhergehenden Ansprüche, **dadurch gekennzeichnet, dass** die Sicherungsscheibe (28) im Verbindungsbereich mit der Halteeinrichtung (22) elastisch ausgebildet ist.

6. Pedikelschraube nach Anspruch 5, **dadurch gekennzeichnet, dass** die elastische Ausbildung eine Abkröpfung ist.

7. Pedikelschraube nach einem der vorhergehenden Ansprüche, **dadurch gekennzeichnet, dass** die Sicherungsscheibe (28) auf die Halteeinrichtung (22) aufklipsbar ist.

8. Pedikelschraube nach einem der vorhergehenden Ansprüche, **dadurch gekennzeichnet, dass** die Sicherungsscheibe (28) mehrere Schlitze (36) aufweist.

9. Pedikelschraube nach Anspruch 8, **dadurch gekennzeichnet, dass** zwischen den Schlitzen (36) Flügel (38) ausgebildet sind.

10. Pedikelschraube nach einem der vorhergehenden Ansprüche, **dadurch gekennzeichnet, dass** die Sicherungsscheibe (28) Öffnungen (34) oder Durchbrüche aufweist, an welchen ein Werkzeug ansetzbar ist.

11. Pedikelschraube nach einem der vorhergehenden Ansprüche, **dadurch gekennzeichnet, dass** die Sicherungsscheibe (28) aus Metall, insbesondere Edelstahl oder Titan oder aus Kunststoff besteht.

## Claims

1. A pedicle screw having a screw shaft (14) with a threading (16) and a screw head (10), with the screw head (10) having a mechanism for affixing correcting elements, and the free end (20) of the screw shaft (14) has an apparatus to prevent it from pulling out, which comprises a lock washer (28) and a holding device (22) for the lock washer (28) that secures the pedicle screw from unintentional detachment after it is screwed in, wherein the lock washer (28) has an opening (32) that receives the holding device (22), **characterized in that** the opening (32) is open on an edge and has a slit (36) discharging into the opening (32).

2. Pedicle screw according to claim 1, **characterized in that** the holding device (22) is independent of the threading (16).

3. Pedicle screw according to one of the preceding claims, **characterized in that** the holding device (22) defines at least one circumferential groove.

4. Pedicle screw according to one of the preceding claims, **characterized in that** the holding is an additional threading (26).

5. Pedicle screw according to one of the preceding claims, **characterized in that** the safety lock washer (28) is structured to be elastic in a connection area with the holding device (22).

6. Pedicle screw according to claim 5, **characterized in that** the elastic structure is a bending.

7. Pedicle screw according to one of the preceding claims, **characterized in that** the lock washer (28) can be snapped onto the holding device (22).

8. Pedicle screw according to one of the preceding claims, **characterized in that** the lock washer (28) defines a plurality of slits (36).

9. Pedicle screw according to claim 8, **characterized in that** wings (38) are positioned between the slits (36).

10. Pedicle screw according to one of the preceding claims, **characterized in that** the lock washer (28) defines openings (34), or holes, in which a tool can engage.

11. Pedicle screw according to one of the preceding claims, **characterized in that** the lock washer (28) is made of metal, in particular, stainless steel or titanium, or out of plastic.

## Revendications

1. Vis pédiculaire, avec une tige de vis (14), présentant un filetage (16), et une tête de vis (10), la tête de vis (10) présentant des dispositifs pour la fixation d'éléments de correction, et l'extrémité (20) libre de la tige de vis (14) étant munie d'une sécurité d'arrachement, qui comprend une rondelle de sécurité (28) et un dispositif de maintien (22) pour la rondelle de sécurité (8), assurant la vis pédiculaire contre tout desserrement intempestif après le vissage, la rondelle de sécurité (28) présentant un passage (32) recevant le dispositif de maintien (22) **caractérisé en ce que** le passage (32) est ouvert est présente une fente (36) débouchant dans le passage (32).

2. Vis pédiculaire selon la revendication 1, **caractérisée en ce que** le dispositif de maintien (22) est indépendant du filetage (16).

3. Vis pédiculaire selon l'une des revendications précédentes, **caractérisée en ce que** le dispositif de maintien (22) est réalisé à la forme d'au moins une cannelure périphérique.

4. Vis pédiculaire selon l'une des revendications précédentes, **caractérisée en ce que** le dispositif de maintien est un autre filetage (26).

5. Vis pédiculaire selon l'une des revendications précédentes, **caractérisée en ce que** la rondelle de sécurité (28) est élastique dans la zone de liaison au dispositif de maintien (22).

6. Vis pédiculaire selon la revendication 5, **caractérisée en ce que** la réalisation élastique est un coudage.

7. Vis pédiculaire selon l'une des revendications précédentes, **caractérisée en ce que** la rondelle de sécurité (28) est susceptible d'être enclipsée sur le dispositif de maintien (22).

8. Vis pédiculaire selon l'une des revendications précédentes, **caractérisée en ce que** la rondelle de sécurité présente plusieurs fentes (36).

9. Vis pédiculaire selon la revendication 8, **caractérisée en ce qu'**entre les fentes (36) sont réalisées des ailettes (38).

10. Vis pédiculaire selon l'une des revendications précédentes, **caractérisée en ce que** la rondelle de sécurité (28) présente des ouvertures (34) ou des passages, sur lesquel(le)s un outil est susceptible d'être appliqué.

11. Vis pédiculaire selon l'une des revendications précédentes, **caractérisée en ce que** la rondelle de sécurité (28) est formée de métal, en particulier d'acier spécial, ou de titane, ou de matière synthétique.
